# EUROPEAN PATENT SPECIFICATION

(11) **EP 3 751 582 B1**
(45) Date of publication and mention of the grant of the patent: **22.05.2024**
(21) Application number: 20179448.4
(22) Date of filing: 11.06.2020
(51) Int. Cl.: G16H 20/40, G16H 50/20, G16H 30/40, A61N 5/10

(54) **RADIOTHERAPY SYSTEM, AND THERAPY PLANNING METHOD**
STRAHLENTHERAPIEANLAGE UND THERAPIEPLANUNGSVERFAHREN
SYSTÈME DE RADIOTHÉRAPIE ET PROCÉDÉ DE PLANIFICATION DE THÉRAPIE

(30) Priority: 13.06.2019 JP 2019110456; 15.05.2020 JP 2020085959
(43) Date of publication of application: 16.12.2020
(73) Proprietor: Canon Medical Systems Corporation, Otawara-shi, Tochigi 324-0036 (JP)
(72) Inventor: KOYAMA, Masanori, Tochigi 324-0036 (JP)
(74) Representative: Marks & Clerk LLP

(56) References cited:
- US-A1- 2018 353 148
- US-A1- 2019 057 504
- US-A1- 2019 114 765
- US-A1- 2019 159 744

## Description

### FIELD

Embodiments disclosed in the present specification, etc. relate to a radiotherapy system, and a therapy planning method.

### BACKGROUND

A site where a first tumor occurs is called a "primary lesion". For example, if a tumor occurs in a stomach first and the tumor cells then metastasize to a lung through blood and lymph circulation, the primary lesion of the metastatic tumor occurring in the lung is determined to be the stomach. What is formed in the body part as a result of the metastasis consists of cells originating from the stomach tumor, and not cells originating from the lung tumor.

When a therapy is planned in radiotherapy, a task of tracing the outline of a tumor using a CT image is performed. This circling of the tumor outline is performed without making a distinction between a primary lesion and a metastatic lesion.

US 2019/159744 relates to the use of computed tomography perfusion (CTP) in a method to identify cancerous lesions having genetic mutations and treat them accordingly. Also, CTP values are used to distinguish primary versus metastatic lesions.

US 2019/057504 relates to an image processor for diagnosing a medical image which comprises a diagnoser which analyzes the medical image using a trained classifier which calculates an index indicating a probability that the medical image corresponds to any of categories of lesion patterns.

### SUMMARY

In a first aspect of the invention, a radiotherapy system is provided as recited in claim 1. In a second aspect of the invention, a therapy planning method is provided as recited in claim 14. The appended dependent claims set out optional features of the invention.

### BRIEF DESCRIPTION OF THE DRAWINGS

FIG. 1 is a diagram showing a configuration of a radiotherapy system according to a first example.
FIG. 2 is a diagram showing a configuration of a therapy planning support apparatus shown in FIG. 1.
FIG. 3 is a diagram showing a typical workflow realized by the execution of a treatment planning support program by the processing circuitry shown in FIG. 2.
FIG. 4 is a diagram schematically showing an input-output relationship of a learned model that classifies a tumor area as either a primary lesion or a metastatic lesion.
FIG. 5 is a diagram of a treatment-planning CT image with markers placed on tumor areas included in the treatment-planning CT image.
FIG. 6 is a diagram showing a whole-body image and slice images.
FIG. 7 is a diagram which shows encircling using a fixed-shape marker.
FIG. 8 is a diagram which shows encircling using a marker tracing an outline of the tumor area.
FIG. 9 is a drawing showing an example of a screen displaying a message recommending the production of a therapeutic plan factoring in the Abscopal effect.
FIG. 10 is a drawing which schematically shows an input-output relationship of another learned model that classifies a tumor area as either a primary lesion or a metastatic lesion.
FIG. 11 is a diagram showing a configuration of a therapy planning apparatus according to a second example.
FIG. 12 is a diagram showing a typical workflow realized by the execution of a treatment planning program by the processing circuitry shown in FIG. 11.
FIG. 13 is a diagram showing a configuration example of a therapeutic plan of a right lung cancer group.
FIG. 14 is a diagram showing a configuration of a therapy planning apparatus according to a third example.
FIG. 15 is a diagram showing a typical workflow realized by the execution of a treatment planning program by the processing circuitry shown in FIG. 14.

### DETAILED DESCRIPTION

In general, according to a first example, a radiotherapy support apparatus has processing circuitry. The processing circuitry specifies at least one tumor area in a medical image of a patient, and outputs classification result information including results of classification of this/these tumor area(s) as either a primary lesion or a metastatic lesion based on a feature amount of this/these tumor areas.

Hereinafter, a radiotherapy system, a therapy planning support method, and a therapy planning method will be exemplarily described below with reference to the accompanying drawings.

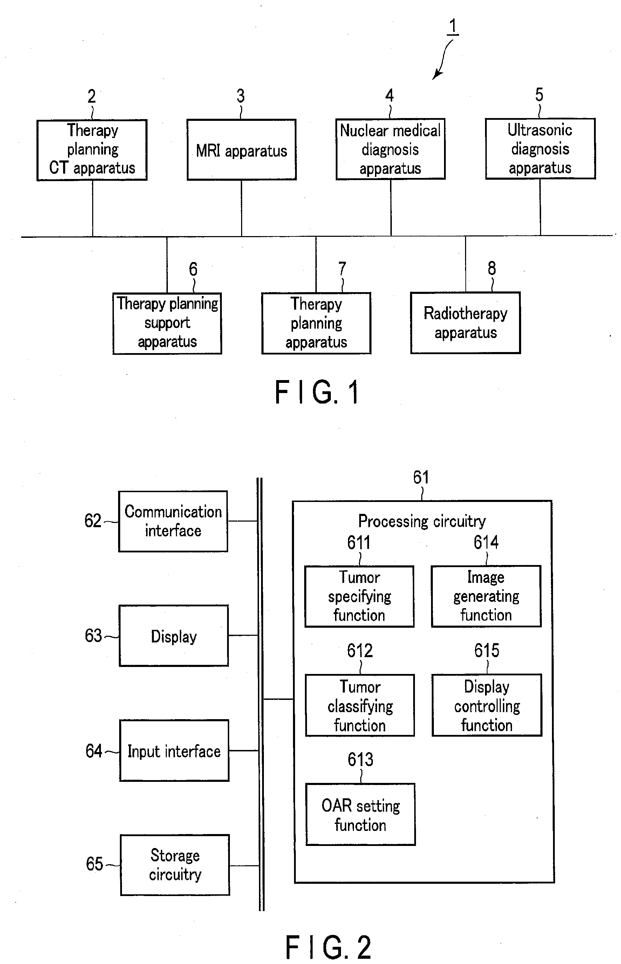

FIG. 1 is a diagram showing a configuration of a radiotherapy system. As shown in FIG. 1, the radiotherapy system 1 includes a therapy planning CT apparatus 2, an MRI (magnetic resonance imaging) apparatus 3, a nuclear medical diagnosis apparatus 4, an ultrasonic diagnosis apparatus 5, a therapy planning support apparatus 6, a therapy planning apparatus 7, and a radiotherapy apparatus 8, which are connected to each other via a network.

The therapy planning CT apparatus 2, the MRI apparatus 3, the nuclear medical diagnosis apparatus 4, and the ultrasonic diagnosis apparatus 5 are medical modalities that generate medical images of a patient.

The therapy planning CT apparatus 2 is an X-ray computed tomography apparatus that generates CT images used in therapy planning. Via rotation of a rotating frame that holds an X-ray tube and an X-ray detector at high speed, the radiotherapy-planning CT apparatus 2 irradiates a patient with X-rays from the X-ray tube, and detects the X-rays passed through the patient using the X-ray detector. The radiotherapy-planning X-ray CT apparatus 2 then generates a CT image expressing a spatial distribution of X-ray attenuation coefficients of substances on an X-ray transmission path, based on raw data from the X-ray detector. The CT image used for therapy planning is called a "therapy-planning CT image".

The MRI apparatus 3 emits, for example, RF pulses from an RF coil to excite target atomic nuclei existing in a patient laying in a static magnetic field, and acquires MR signals generated from the target atomic nuclei using the RF coil. The MRI apparatus 3 then generates an MR image expressing the spatial distribution of the target atomic nuclei based on the MR signals from the RF coil.

The nuclear medical diagnosis apparatus 4 generates a nuclear medical diagnosis image expressing the spatial distribution of radioactive nuclides accumulated in a patient. As the nuclear medical diagnosis apparatus 4, a PET (positron emission tomography) apparatus or a SPECT (single photon emission CT) apparatus is used, for example. The PET apparatus counts a pair of 511 keV gamma rays generated upon pair annihilation of each of the positrons generated from radionuclides accumulated in a patient and the corresponding electron existing around the radionuclides through use of coincidence circuitry, thereby generating a PET image expressing the spatial distribution of the radionuclides based on coincidence count signals from the coincidence circuitry. The SPECT apparatus detects single photon gamma rays generated from radionuclides accumulated in a patient by using a radiation detector to generate a SPECT image expressing the spatial distribution of the radionuclides based on the radiation detector detection signals.

The ultrasonic diagnosis apparatus 5 transmits ultrasonic waves into a patient with the ultrasonic probe, and receives ultrasonic waves reflected in the body of the patient with the ultrasonic probe, thereby generating an ultrasonic image through performance of signal processing on echo signals corresponding to the received ultrasonic waves. For the signal processing, B-mode processing or Doppler processing is used, for example. The ultrasonic diagnosis apparatus 5 performs the B-mode processing on the echo signals to generate a B-mode image expressing the spatial distribution of acoustic impedance in the patient, and performs the Doppler processing on the echo signals to generate a Doppler waveform or a Doppler image expressing kinetic information of tissue.

Hereinafter, MR images generated by the MRI apparatus 3, nuclear medical images generated by the nuclear medical diagnosis apparatus 4, and ultrasonic images generated by the ultrasonic diagnosis apparatus 5 will be collectively referred to as "other-apparatus images" so as to distinguish them from a treatment-planning CT image. Suppose the treatment-planning CT image and the other-apparatus images are volume data constituted by a plurality of voxels arranged three-dimensionally. Suppose at least one tumor occurs in the body of a patient in the present example.

In other words, at least one image area relating to a tumor (hereinafter "tumor area") exists in each of the treatment-planning CT image and the other-apparatus images.

The therapy planning support apparatus 6 is a computer that aids in therapy planning by the therapy planning apparatus 7. Specifically, the therapy planning support apparatus 6 specifies tumor areas present in the patient's body by making a distinction between a primary lesion and a metastatic lesion, using the therapy-planning CT image and the other-apparatus images, and places markers against the specified tumor areas. The therapy-planning CT image in which markers are placed against the tumor areas is supplied to the particle therapy planning apparatus 7. The details of the therapy planning support apparatus 6 will be described later.

The therapy planning apparatus 7 is a computer including a processor such as a CPU, a memory such as an ROM or an RAM, a display, an input interface, and a communication interface. The particle therapy planning apparatus 7 is a computer that produces a therapeutic plan for the patient by using the therapy-planning CT image in which markers are placed against the tumor areas. As a method of producing a therapeutic plan, a forward planning method and an inverse planning method are known. With the forward planning method, radiotherapy conditions, such as the number of irradiation directions, each irradiation angle, irradiation intensity of each irradiation, a collimator opening degree of each irradiation, and a wedge filter, etc., are set in detail, and these conditions are then evaluated based on an irradiation distribution obtained under these conditions. To change the radiation distribution, all or some of the radiotherapy conditions are changed, and the radiation distribution is once again calculated. Thus, in the forward planning method, the radiation distribution is gradually changed through repeatedly changing the radiotherapy conditions, until a desired radiation distribution is obtained. With the inverse planning method, a tumor area and an appropriate margin thereof are set, a radiation dose to irradiate the area, and a permissible range of the radiation dose are set. In addition, an organ-at-risk (OAR) area is extracted from the medical image, and a radiation dose for the OAR area is set at a safety level that does not exceed a certain level of radiation dose.

The radiotherapy apparatus 8 is an apparatus that treats a patient by irradiating a target tumor, etc. present in the patient with radiation in accordance with a radiotherapy plan. Specifically, the radiotherapy apparatus 8 has a treatment gantry, a treatment bed, and a console. The treatment gantry supports an irradiation head rotatably around a rotation axis. In the irradiation head are installed an accelerator tube that accelerates electrons, etc. generated from an electron gun, etc., and a metal target against which the electrons accelerated by the accelerator tube collide. X-rays, which are a type of radiation, are generated by the electrons colliding with the metal target. The irradiation head performs irradiation of radiation in accordance with the therapeutic plan made by the therapy planning apparatus 7. The treatment bed includes a treatment top plate on which a patient is laid, and a base that supports the treatment top plate in a freely movable manner. The treatment gantry, the treatment bed, and the patient are aligned so that the patient's body part targeted for treatment corresponds to an isocenter.

FIG. 2 is a diagram showing a configuration of the therapy planning support apparatus 6 shown in FIG. 1. As shown in FIG. 2, the radiotherapy planning apparatus 6 includes processing circuitry 61, a communication interface 62, a display 63, an input interface 64, and storage circuitry 65.

The processing circuitry 61 includes, as hardware resources, a processor such as a CPU (Central Processing Unit) or a GPU (Micro Processing Unit) and memories such as a ROM (Read Only Memory) and a RAM (Random Access Memory). The processing circuitry 61 executes a program relating to the therapy planning support (hereinafter, referred to as "the therapy planning support program"), to enable a tumor specifying function 611, a tumor classifying function 612, an OAR setting function 613, an image generating function 614, and a display controlling function 615.

The processing circuitry 61 that enables the tumor specifying function 611 specifies at least one tumor area in the therapy-planning CT image relating to a patient. The processing circuitry 61 may specify at least one tumor area in the other-apparatus images relating to the same patient.

The processing circuitry 61 that enables the tumor classifying function 612 classifies this/these tumor area(s) as either a primary lesion or a metastatic lesion based on a first pixel value regarding this/these tumor area(s) included in the therapy-planning CT image, and a second pixel value regarding this/these tumor area(s) included in the other-apparatus images. A primary lesion is a site where a tumor first occurs. A metastatic lesion is a site where tumor cells of a primary lesion is metastasized. The first pixel value and the second pixel value are examples of image feature amount.

The processing circuitry 61 that enables the OAR setting function 613 sets an image area corresponding to an OAR (hereinafter, "OAR area") in the therapy-planning CT image. The processing circuitry 61 may set an OAR area in the other-apparatus images as well.

The processing circuitry 61 that enables the image generating function 614 performs three-dimensional image processing on the three-dimensional therapy-planning CT image to generate a two-dimensional therapy-planning CT image. The processing circuitry 61 also performs three-dimensional image processing on three-dimensional other-apparatus images to generate two-dimensional other-apparatus images. For the three-dimensional imaging processing, rendering techniques, such as volume rendering, surface rendering, pixel value projection processing, MPR (Multi-Planer Reconstruction), CPR (Curved MPR), etc., may be adopted.

The processing circuitry 61 that enables the display control function 615 displays various types of information via the display 63. Specifically, the processing circuitry 61 displays the two-dimensional therapy-planning CT image and the two-dimensional other-apparatus images generated by the image generating function 614 via the display 63. At this time, the processing circuitry 61 displays the tumor area(s) in the two-dimensional therapy-planning CT image and the two-dimensional other-apparatus images in a manner that renders the primary lesion and the metastatic lesion visually distinguishable.

The communication interface 62, via wires (not shown) or wirelessly, performs a data communication with the therapy planning CT apparatus 2, the MRI apparatus 3, the nuclear medical diagnosis apparatus 4, the ultrasonic diagnosis apparatus 5, the therapy planning apparatus 7, and the radiotherapy apparatus 8, which constitute the radiotherapy system 1.

The display 63 displays various types of information through the display controlling function 615. As the display 63, it is possible to use, for example, a CRT display, a liquid crystal display, an organic EL display, an LED display, a plasma display, or an arbitrary display known in this technical field. The display 63 may be a projector.

The input interface 64 includes an input device and input interface circuitry. The input device receives various types of instructions from a user. A keyboard, a mouse, or switches etc. may be used as the input device. The input interface circuitry supplies an output signal from the input device to the processing circuitry 61.

The storage circuitry 65 is a storage device which stores various types of information, such as an HDD (hard disk drive), an SSD (solid state drive), or integrated circuit storage. The storage circuitry 65 may also be a drive assembly or the like which reads and writes various types of information to and from portable storage media, such as a CD-ROM drive, a DVD drive, or a flash memory. For example, the storage circuitry 65 stores the therapy planning support program, the therapy-planning CT images, and the other-apparatus images, etc.

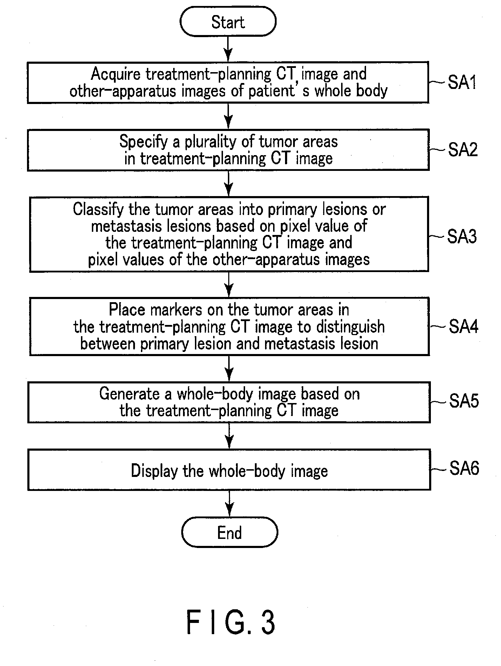

An example of the operation of the radiotherapy planning apparatus 6 will be described below. FIG. 3 is a diagram showing a typical workflow realized through execution of the treatment planning support program by the processing circuitry 61.

First, the processing circuitry 61 acquires a therapy-planning CT image and other-apparatus images of the patient's whole body (step SA1). The therapy-planning CT image and the other-apparatus images are the images showing the same patient's whole body as an imaging target, and are volume data. As the other-apparatus images, images generated by a different principle of imaging from that of the therapy-planning CT image may be used. For example, as the other-apparatus images, a nuclear medical diagnosis image obtained by the nuclear medical diagnosis apparatus, a Doppler image obtained by the ultrasound diagnosis apparatus, and an MR image obtained by the MRI apparatus are preferable, supposing multiple tumors occur in the patient who is a target of the therapeutic plan. An image of the patient's whole body acquired herein is not necessarily a CT image. It may be an other-apparatus image (e.g., a nuclear medical diagnosis image). If the image of the patient's whole body is a nuclear medical diagnosis image, the other-apparatus image may be a CT image or an MR image.

After step SA1 is performed, the processing circuitry 61 that enables the tumor specifying function 611 specifies a plurality of tumor areas in the therapy-planning CT image acquired in step SA1 (step SA2). In step SA2, the processing circuitry 61 extracts a plurality of tumor areas in the therapy-planning CT image though imaging processing. As the imaging processing, threshold processing using, as thresholds, upper and lower limits of the CT values that the tumor areas may have, may be performed, for example. As other types of image processing, tumor extraction processing using anatomical knowledge, or tumor extraction processing through machine learning may be adopted. Since the three-dimensional images of the patient's whole body are a target of the extraction processing as described above, it is possible to extract all tumor areas even in a case where tumors are spreading throughout the body. Furthermore, the expectation is that tumor areas may be extracted without fail by the automatic extraction of tumor areas through the image processing. An image area designated by the user via the input interface 64 may be extracted as a tumor area.

In step SA2, the processing circuitry 61 may extract a plurality of tumor areas in the therapy-planning CT image based on the therapy-planning CT image and the other-apparatus images. Specifically, the processing circuitry 61 positions the therapy-planning CT image and the other-apparatus images, and generates a synthesized image (fusion image) of the therapy-planning CT image and the other-apparatus images after the positioning. Next, the processing circuitry 61 transfers the tumor areas included in the other-apparatus images to the therapy-planning CT image. Specifically, the processing circuitry 61 extracts a plurality of tumor areas through performance of the imaging processing on the other-apparatus images, specifies the locations of the tumor areas in the other-apparatus images, and sets locations in the therapy-planning CT image at the same coordinates as the specified locations. For example, a pixel value of the nuclear medical diagnosis image, which is one of the other-apparatus images, reflects a function of tissue such as metabolism or activity, and therefore, the nuclear medical diagnosis image differs from the therapy-planning CT image in which a morphological index such as a CT value is a pixel value. There is a case where a tumor not shown in the therapy-planning CT image is instead shown in the other-apparatus images, such as the nuclear medical diagnosis image. Accordingly, through performing the above-described transcription, it is possible to specify tumor areas thoroughly.

After step SA2 is performed, the processing circuitry 61 that enables the tumor classifying function 612 classifies each tumor area specified in step SA2 as either a primary lesion or a metastatic lesion based on the pixel value of the therapy-planning CT image and the pixel values of the other-apparatus images (step SA3). The pixel value of the therapy-planning CT image and the pixel values of the other-apparatus images are examples of image feature amounts. A lethal amount of tumor may differ depending on a tumor type; accordingly, it is important to classify a tumor area as either a primary lesion or a metastatic is lesion when a therapeutic plan is produced. For example, if a tumor first occurs in a stomach, and the tumor cells metastasize to a lung through blood and lymph circulation, the primary lesion of the metastatic tumor in the lung is a stomach tumor. The tumor that occurs in the body part as a result of the metastasis consists of cells originating from the stomach tumor, and not cells originating from the lung tumor. In this case, the tumor area is classified as a metastatic lesion by the tumor classifying function 612.

In step SA3, the processing circuitry 61 specifies a location and a type of a tumor based on the image feature amounts regarding a tumor area, and classifies the tumor area as either a primary lesion or a metastatic lesion in accordance with a result of the specification. For example, if the location of the tumor is a left lung, and the type of the tumor is stomach cancer, the tumor or the tumor area is classified as a metastatic lesion.

In step SA3, the processing circuitry 61 may perform the classification of the tumor areas as either a primary lesion or a metastatic lesion through the use of machine learning. For the algorithm of the machine learning, linear classifiers such as logistic regression and support vector machine, or non-linear algorithms, such as neural network, random tree, and random forest, etc., may be used. Hereinafter, suppose the algorithm of the machine learning is a deep neural network (DNN), which is a type of neural network. The DNN is defined by a parameterized synthesis function defined by a combination of a plurality of adjustable functions and parameters. Weighting coefficients and parameters are collectively called "the parameters".

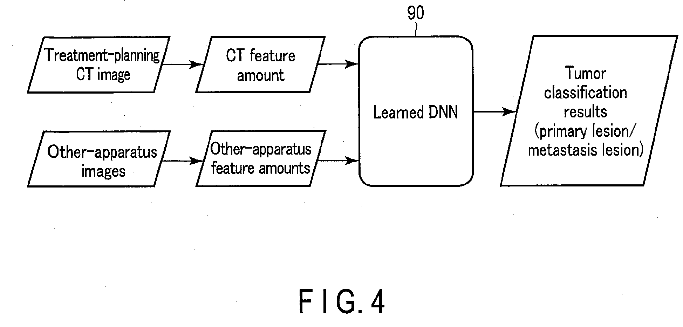

FIG. 4 is a diagram schematically showing an input-output relationship of the learned DNN 90 that classifies a tumor area as either a primary lesion or a metastatic lesion. The learned DNN 90 is a DNN into which the feature amount of the therapy-planning CT image and the feature amounts of the other-apparatus images are input and learned so as to output classification result information including a result of classification of a tumor area as either a primary lesion or a metastatic lesion. The learned DNN 90 has input layers, intermediate layers, and output layers. The intermediate layers include a CNN (convolutional neural network) and fully connected layers. The intermediate layers preferably include at least two layers.

The processing circuitry 61 inputs image feature amounts relating to a tumor area of the patient into the learned DNN 90. To the input layers, the feature amount of the therapy-planning CT image (hereinafter "the CT feature amount") and the feature amounts of the other-apparatus images (hereinafter "the other-apparatus feature amounts") are input. The CT feature amount is calculated based on the therapy-planning CT image, and the other-apparatus feature amounts are calculated based on the other-apparatus images. The processing circuitry 61 calculates a CT feature amount for each of the plurality of tumor areas included in the therapy-planning CT image, and calculates the other-apparatus feature amounts for each of the plurality of tumor areas included in the other-apparatus images. The CT feature amount and the other-apparatus feature amounts are information for specifying a location of tumor (organ to which a tumor belongs) and a type of tumor). The information may be, for example, a coordinate indicating a location of a tumor area in a medical image, a name or a symbol of an anatomical site where a tumor is present, a pixel value of a tumor area, a statistic value of a pixel value, and a morphological feature amount. The pixel value of a tumor area may include not only a pixel value indicating a tumor area but also a pixel value of the vicinity of the tumor area. The statistic value of pixel value is at least one of the following feature amounts relating to statistic values: an average, a maximum value, a minimum value, an intermediate value, standard deviation, a dispersion value, etc. of pixel values of a plurality of pixels constituting the tumor area. The morphological feature amount is at least one of the following feature amounts relating to forms: a volume, a surface area, the number of hollows, a degree of shape, etc. of a tumor area. The degree of shape is at least one of the following feature amounts relating to shapes: a degree of roundness, sharpness, the number of projections and recesses, etc. of a tumor area. The processing circuitry 61 specifies a type of tumor based on, for example, a pixel value of a tumor area, a statistic value of a pixel value, or a morphological feature amount. The type of tumor is information relating to an anatomical site of a primary lesion, such as stomach cancer and lung cancer, etc. The processing circuitry 61 also specifies a location of tumor based on a coordinate indicating a location of a tumor area, and a name or a symbol of an anatomical site where a tumor is present. The coordinate indicating the location of the tumor area may be a coordinate of a representative point, such as a central point, a gravity point, a feature point, or a clinical interest point of the tumor area, or a coordinate of each pixel constituting the tumor area, or a coordinate of an edge point or a boundary pixel of the tumor area (a coordinate of a pixel indicating an outline of the tumor area).

The types of the CT feature amount and the other-apparatus feature amounts input into the input layers are not limited to a single type, and multiple types may be input. For example, for each tumor area, all of a coordinate indicating a location of a tumor area, a name or a symbol of an anatomical site where a tumor is present, a pixel value of the tumor area, a statistic value of pixel value, and a morphological feature amount may be input, or information of selectively combined those values may be input. The learned DNN 90 may be a learned DNN into which only information specifying a tumor type (at least one of a pixel value of a tumor area, a statistic value of a pixel value, or a morphological feature amount) as the feature amount of the therapy-planning CT image and the feature amounts of the other-apparatus images, and learned so as to output a tumor type. In this case, the processing circuitry 61 obtains classification result information including a result of the classification of a tumor area as either a primary lesion or a metastatic lesion based on the tumor type calculated by the learned DNN 90 and a result of the specific processing performed in step SA2. Furthermore, the other-apparatus feature amounts input into the input layers are not limited to the feature amounts based on one of the other-apparatus image types, and multiple types of feature amounts based on more than one other-apparatus image may be input to the input layers. The pixel values have different physical meanings, depending on a principle of imaging, etc. of the medical image diagnosis apparatus that generates the image. For example, the pixel value of the therapy-planning CT image represents an X-ray attenuation coefficient of tissue; the pixel value of the MRI apparatus represents a hydrogen nucleus density; the pixel value of the nuclear medical diagnosis image represents a concentration of an agent accumulated in tissue (in other words, metabolism or activity of tissue); and the pixel value of a Doppler image obtained by an ultrasonic diagnosis apparatus represents movement information of tissue (for example, blood flow moving subject). Through the use of the feature amounts based on various types of images, it is possible to improve accuracy in the classification of tumor areas as either a primary lesion or a metastatic lesion.

To the learned DNN 90, image data of each tumor area included in a therapy-planning CT image may be input as the CT feature amounts, and image data of each tumor area included in other apparatus images may be input as the other-apparatus feature amounts. The image data of each tumor area may be extracted by discretionarily-selected image recognition processing, etc. by the processing circuitry 61, or may be extracted from a range designate by a user via the input interface 64, etc. Each tumor area may be extracted along the outline of the tumor area, or an image area including an excessive area around the tumor area may be extracted.

Classes of the classification result are set as "primary lesion" and "metastatic lesion". For example, the classification result includes a probability that a tumor area is a primary lesion and a probability that a tumor area is a metastatic lesion. For the output layers of the learned DNN 90 for outputting the classification result, for example a Max function or a soft Max function may be used.

For example, for each tumor area included in the therapy-planning CT image, the learned DNN 90 receives an input of a CT feature amount and other-apparatus feature amounts relating to each given tumor area, and outputs a classification result of the tumor area. If the probability that the tumor area is a primary lesion is higher than the probability that the tumor area is a metastatic lesion, the processing circuitry 61 allocates a flag indicating that the tumor area is a primary lesion (hereinafter "primary lesion flag") to the tumor area; if the probability that the tumor area is a metastatic lesion is higher than the probability that the tumor area is a primary lesion, the processing circuitry 61 allocates a flag indicating that the tumor area is a metastatic lesion (hereinafter "metastatic lesion flag") to the tumor area. The processing circuitry 61 repeats the same processing on all tumor areas; as a result, the primary lesion flag or the metastatic lesion flag is allocated to each tumor area.

The generation of the learned DNN 90 is performed by the processing circuitry 61 of the therapy planning support apparatus 6, for example. The processing circuitry 61 learns a DNN, through supervised machine learning, on input data of the feature amount of the therapy-planning CT image and the feature amounts of the other-apparatus images, based on training data including correct answer data, which is a correct result of the classification as either a primary lesion or a metastatic lesion. The training data is collected for various tumor areas of various patients. For example, the processing circuitry 61 performs forward propagation by applying a DNN to a single CT feature amount and the other-apparatus feature amounts, and outputs an estimated classification result. Next, the processing circuitry 61 performs backpropagation by applying the machine learning model to a difference (error difference) between the estimated classification result and the correct classification result, and calculates a gradient vector. Subsequently, the processing circuitry 61 updates parameters of the DNN, such as a weighted matrix and a bias, etc. based on the gradient vector. The learned DNN 90 is completed through repeating the forward propagation and the backpropagation on a number of sets of training data so as to update the parameters. The learned DNN 90 may be generated by other computers.

To improve accuracy of the tumor classification, additional information other than the CT feature amount and the other-apparatus feature amounts may be input into the learned DNN 90. Appropriate additional information is patient basic information, living body measurement values, genetic information, finding information, etc. of the patient. As the patient basic information, a two-dimensional image with respect to a sagittal cross section or a coronal cross section is generated by performing MPR processing on a three-dimensional therapy-planning CT image. Age, sex, height, weight, and the like may be used. As the living body measurement values, a blood composition, an electrocardiogram waveform, a respiration waveform, and measurement values obtained by respiration test, urinalysis, and stool test may be used. The finding information is information regarding a physician's findings on the therapy-planning CT image or other-apparatus images, etc.

The above classification processing may be modified in various ways. For example, processing of obtaining the CT feature amount and the other-apparatus feature amounts from a medical image may be incorporated into the learned DNN 90. In the above learned DNN 90, it is assumed that the CT feature amount and other-apparatus feature amounts of each tumor area are input, and a classification result of the tumor area is output. However, the present embodiment is not limited thereto. All, or two or more, tumor areas included in a single therapy-planning CT image and other-apparatus images are input, and a classification result may be output for each of the tumor areas in all, or two or more, sets of the tumor areas that have been input. The classification result is not necessarily a combination of a probability that a tumor area is a primary lesion and a probability that a tumor area is a metastatic lesion; rather, it may be either of them, or an identifier of either of the classes with a higher probability may be output. Furthermore, the classification result is not necessarily a probability; rather, any index that can express a probability that a tumor area is either a primary lesion or a metastatic lesion may be output. The classes are not limited to two classes consisting of "primary lesion" and "metastatic lesion"; however, they may be three or more classes consisting of primary lesion, metastatic lesion, and other classes.

The method of classification as either a primary lesion or a metastatic lesion is not limited to the method using machine learning. Techniques such as data integration and data mining may be adopted. For example, an empirical or logical relational expression that derives a classification as either a primary lesion or a metastatic lesion from the CT feature amount and the other-apparatus feature amounts may be adopted. In this case, the processing circuitry 61 applies the relational expression to the pixel value of the therapy-planning CT image and the pixel values of the other-apparatus images, and classifies each tumor area as either a primary lesion or a metastatic lesion.

In step SA3, the processing circuitry 61 that enables the tumor classifying function 612 outputs classification result information for each tumor area. The classification result information may be information obtained by the processing circuitry 61 in accordance with specific results of a tumor location and a tumor type obtained in step SA2, or may be information obtained by the learned model 90. The classification result information includes identification information, location information, and primary lesion information for each tumor area. The identification information is information indicating whether the tumor area is a primary lesion or a metastatic lesion. For example, the identification information includes the primary lesion flag indicating that the tumor area is a primary lesion, and the metastatic lesion flag indicating that the tumor area is a metastatic lesion. The location information is information relating to a location of a body part where the tumor area occurs. For example, the location information is a name or a symbol of an organ to which a tumor belongs (an anatomical part of the tumor-occurring part), or a coordinate indicating a location of a tumor area in the therapy-planning CT image. The primary lesion information is information indicating if the tumor area is classified as a primary lesion, in this regard, and is information specifying a primary lesion if the tumor area is classified as a metastatic lesion. Information that specifies a primary lesion may be location information of a primary lesion or an identifier placed against a tumor area which is a primary lesion. The location information of a primary lesion is, as described above, a name or a symbol of an anatomical site of a primary lesion, or a coordinate indicating a location of a tumor area in a therapy-planning CT image. The name or symbol of an anatomical site of a primary lesion can be indicated based on a tumor type specified by the learned DNN 90, even when a primary lesion is not included in a medical image. For example, if stomach cancer is specified in a left lung by the learned DNN 90, the tumor area is classified as a metastatic lesion, and the name of the anatomical site of the metastatic lesion is categorized into "stomach". On the other hand, the coordinate of a metastatic lesion is indicated only when a metastatic lesion is included in a medical image.

After step SA3, the processing circuitry 61 places a marker that distinguishes between primary lesion and metastatic lesion against each tumor area in the therapy-planning CT image (step SA4).

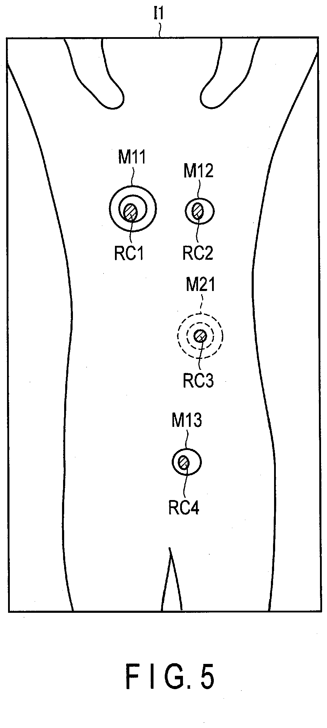

FIG. 5 is a diagram of a treatment-planning CT image I1, with markers M1m and M2p placed to tumor areas RCn included in the treatment-planning CT image. The symbols n, m, and p represent tumor area numbers. For the sake of explanation, the therapy-planning CT image I1 shown in FIG. 5 is a part of a whole-body image with respect to the AP direction. The processing circuitry 61 places markers M1m and M2p, which trace the outline of the tumor area RCn, to each of the tumor areas RCn included in the therapy-planning CT image, based on the classification result information output in step SA3. The markers M1m and M2p shown in FIG. 5 are in the shape of circle; however, any shape, such as a triangle or square, will suffice as the tumor area RCn can be encircled. The process of placing markers M1m and M2p against the tumor area RCn may be referred to as "encircling".

At this time, the processing circuitry 61 places different markers against a tumor area corresponding to a primary lesion and a tumor area corresponding to a metastatic lesion. In other words, different attributes are allocated to the tumor area markers for a primary lesion and the tumor area markers for a metastatic lesion. As the attributes of such markers, colors, shapes, or thickness may be used. For example, as shown in FIG. 5, single-circle markers M12 and M13 are placed against the metastatic lesions RC2 and RC4, and double-circle markers M11 and M21 are placed against the primary lesions RC1 and RC3. The processing circuitry 61 may classify the primary lesion markers of in accordance with types of primary lesion. The attributes of the markers differing according to primary lesions may be any attributes, as long as the attributes are different from those for the markers distinguishing between a primary lesion and a metastatic lesion. For example, as shown in FIG. 5, if the attribute of the markers differing according to whether they are placed against a primary lesion or a metastatic lesion is multiplicity of lines, the attribute of the markers placed against to primary lesions is set to a line type. For example, if the primary lesion is a lung, the solid-line markers M11, M12, and M13 will be placed, and if the primary lesion is a stomach, the broken-line marker M12 will be placed.

After step SA4, the processing circuitry 61 that enables the image generating function 614 generates a whole-body image based on the therapy-planning CT image (step SA5). Specifically, the processing circuitry 61 performs MPR processing on the three-dimensional therapy-planning CT image marked in step SA4, and generates a two-dimensional image with respect to a sagittal cross section or a coronal cross section. The two-dimensional image is a whole-body image, since it covers the entire range of the patient with respect to the body axis. The whole-body image may be a two-dimensional image generated as a result of performing pixel value projection processing, such as MIP, etc., on a three-dimensional therapy-planning CT image, or a two-dimensional image generated as a result of volume rendering processing. The whole-body image may be a two-dimensional image generated by the performance of the three-dimensional imaging on the three-dimensional other-apparatus images, or a two-dimensional image generated based on a synthesis image of the therapy-planning CT image and the other-apparatus images. Thus, it is possible to represent almost all the tumor areas present in the patient's whole body in one piece of whole-body image.

After step SA5, the processing circuitry 61 displays the marked whole-body image (step SA6). In step SA6, the processing circuitry 61 displays for example the whole-body image shown in FIG. 5 via the display 63. Through displaying the tumor areas in the whole-body image, it is possible to improve browsability of the tumor areas, compared to the case where the tumor areas are displayed in an axial cross section image (slice image). Furthermore, through the use of different markers for primary lesion and metastatic lesion, it is possible to identify the primary lesion and the metastatic lesion at a glance. Through the use of different markers according to primary lesion, it is possible to know primary lesion of each tumor area at a glance.

The method of displaying the tumor areas is not limited to the above-described method. For example, a slice image may be displayed along with, or separately from, the whole-body image. The processing circuitry 61 generates, based on the whole-body image, a slice image regarding an axial cross section (slice) that includes one or more tumor areas included in the same slice among at least one tumor area included in the whole-body image.

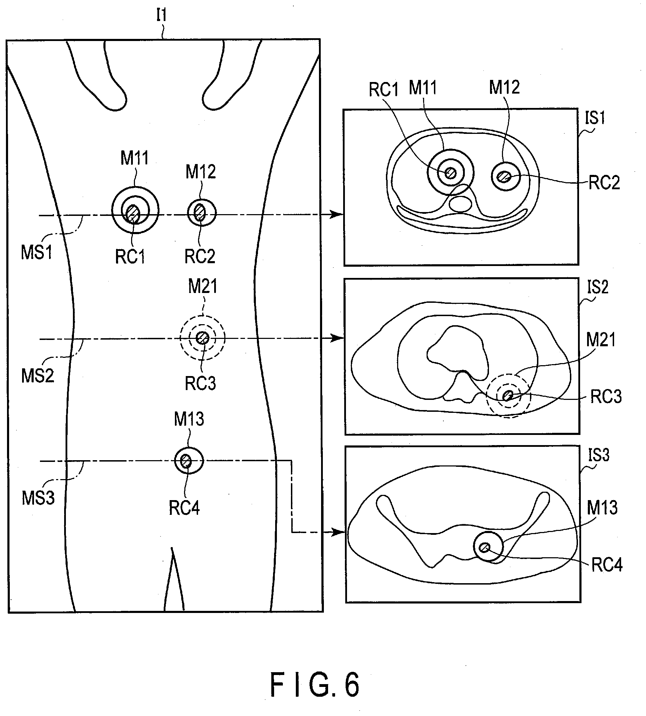

FIG. 6 is a diagram showing the whole-body image I1 and the slice image ISq (q is a slice number). As shown in FIG. 6, the slice image ISq is generated from the whole-body image I1 for each slice position where the tumor area RCn is present. Tumor areas included in each slice image have the same marker as that placed against the whole-body image. The processing circuitry 61 preferably displays the whole-body image and the plurality of slice images side by side on one screen. In this case, the shape indicating the slice position (hereinafter, "slice position marker") MSq (q is a slice number) is preferably placed in the whole-body image so that the slice position corresponding to each slice image can be checked on the whole-body image. In this case, a slice image ISq corresponding to the slice position marker MSq is preferably displayed at the position corresponding to the slice position marker. The slice of the slice image may be a slice having no thickness, or a slice having a thickness.

The processing circuitry 61 may selectively display the whole-body image and the plurality of slice images. For example, if a tumor area of interest included in the whole-body image is designated via the input interface 64, the processing circuitry 61 may display a slice image corresponding to the designated tumor area. If a slice position marker of interest included in the whole-body image is designated via the input interface 64, the processing circuitry 61 may display a slice image corresponding to the designated slice position marker.

The above is the description of an operation example of the therapy planning support.

The therapy-planning CT image marked in step SA4 is transferred to the particle therapy planning apparatus 7. The particle therapy planning apparatus 7 produces a therapeutic plan for the patient based on the marked therapy-planning CT image. According to the present embodiment, a therapeutic plan is produced for each tumor area classified as either a primary lesion or a metastatic lesion based on the therapy-planning CT image reflecting the whole body of the patient. In other words, compared to the case where a therapeutic plan is produced based on a slice image of a specific body part, the production of a therapeutic plan based on a whole-body image allows prompt and easy discovery of a metastatic lesion, which is often difficult to spot, and reduces oversights of metastatic lesion. Since each tumor area is classified as either a primary lesion or a metastatic lesion, and a marker corresponding to a primary lesion or a metastatic lesion is placed against each tumor area, it is possible to produce a therapeutic plan suitable for a primary lesion or a metastatic lesion. Furthermore, since the attributes of the markers are classified according to a primary lesion, it is possible to produce a therapeutic plan in accordance with a type of primary lesion.

A tumor area is encircled based on the therapy-planning CT image which reflects the whole body of the patient; therefore, it is possible to circle the outlines of multiple tumors spreading over the patient's whole body at the same time, and to shorten a time required for circling the outline.

The above-described operation example shown in FIG. 3 may be modified in various ways. For example, in step SA4, the processing circuitry 61 performs the encircling of a tumor while considering an OAR area.

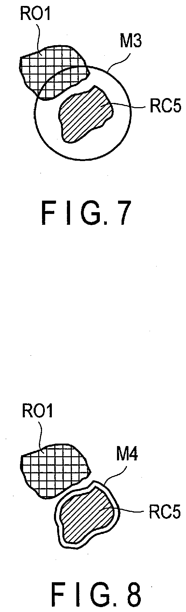

FIG. 7 is a diagram showing the encircling using the fixed-shape marker M3, and FIG. 8 is a diagram showing encircling using the marker M4 that traces an outline of the tumor area. As shown in FIGS. 7 and 8, the tumor area RC5 and the OAR area RO1 are included in the therapy-planning CT image or the other-apparatus images. The tumor area RC5 is specified by the enabled tumor specifying function 611, and the OAR area RO1 is specified by the enabled OAR setting function 613. The processing circuitry 61 specifies an OAR area included in a medical image, and sets a shape of marker so that a marker indicating a location of a tumor area does not overlap the OAR area. The processing circuitry 61 causes the display 63 to display the set marker and the OAR area. If the tumor area RC5 and the OAR area RO1 are in such proximity as shown in FIG. 7, when the tumor area RC5 is encircled by the circle-shaped marker M3, at least a part of the OAR area RO1 may superimpose on the marker M3. If the OAR area is superimposed on a marker, a therapeutic plan that administers a large amount of dose to the OAR area may be produced.

As shown in FIG. 8, the processing circuitry 61 places the marker M5, that traces the outline of the tumor area RC5, against the tumor area RC5. The marker M5 may be set to a size corresponding either to the outline of the tumor area RC5 or to an enlarged outline of the tumor area RC5 by a predetermined magnifying ratio. It is thereby possible to avoid superimposition of the marker M5 on the OAR area RO1, and to reduce the possibility of producing a therapeutic plan to administer a large dose amount to the OAR area RO1.

The processing circuitry 61 may provide support in the production of a therapeutic plan in accordance with whether a tumor area is a primary lesion or a metastatic lesion. For example, an Abscopal effect using an immune system has been receiving attention. The Abscopal effect is a phenomenon where an irradiation of radiation on a primary lesion also affects a metastatic lesion which is not directly radiated. The processing circuitry 61 determines whether or not a criterion for recommending the production of a therapeutic plan in consideration of the Abscopal effect is satisfied by the primary lesion and the metastatic lesion (hereinafter "Abscopal effect criterion"). When it is determined that the Abscopal effect criterion is satisfied, the processing circuitry 61 displays a message recommending the production of a therapeutic plan taking the Abscopal effect into consideration. The Abscopal effect criterion is set to determine, for example, whether a plurality of metastatic lesions are present in relation to a single primary lesion, whether a distance between a primary lesion and its metastatic lesion is greater than a threshold, and the like. In other words, the processing circuitry 61 determines, if there are multiple metastatic lesions in relation to a single primary lesion, to display a message recommending the production of a therapeutic plan taking the Abscopal effect into consideration. The processing circuitry 61 may determine, if the distance between a primary lesion and its metastatic lesion is greater than a threshold, to display a message recommending the production of a therapeutic plan taking the Abscopal effect into consideration.

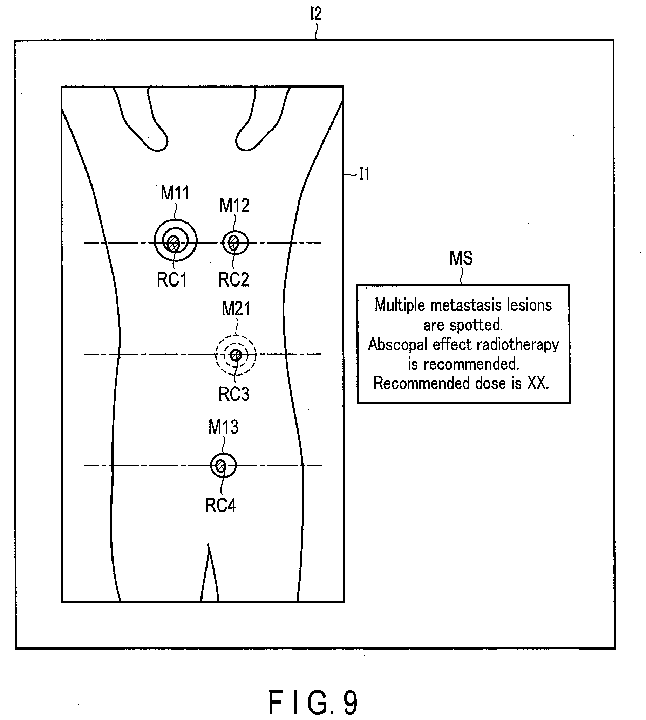

FIG. 9 is a drawing showing an example of the screen I2 displaying a message MS recommending the production of a therapeutic plan factoring in the Abscopal effect. As shown in FIG. 9, in this example, a plurality of metastatic lesions originating from a primary lesion as lung cancer are present, and the Abscopal effect criterion is satisfied. In this case, the processing circuitry 61 displays the message MS recommending the production of a therapeutic plan factoring in the Abscopal effect. The message MS is, for example, "A number of metastatic lesions are spotted. Radiotherapy factoring in the Abscopal effect is recommended". Thus, a user can have an opportunity to produce a therapeutic plan for radiotherapy factoring in the Abscopal effect. For example, as shown in FIG. 9, it is possible to make a therapy strategy, such as a plan consisting of radiotherapy for the primary lesion of lung cancer, observation of two metastatic lesions of the lung cancer, and medication for the primary lesion of stomach cancer.

The processing circuitry 61 may display a recommended dose for all the tumors present in the patient. The recommended dose of the radiotherapy factoring in the Abscopal effect is set to a dose lower than a total dose of the radiotherapy in which a primary lesion and metastatic lesions are independently irradiated with radiation (hereinafter, "original dose") by a predetermined amount. The predetermined amount may be automatically set, or may be designated by the user through the input interface 64. The original dose may be calculated based on a total volume of multiple tumors, a type of primary lesion, biological characteristics of the other tumors, etc.

The processing circuitry 61 may display a recommended dose in the case where the Abscopal effect is factored in radiotherapy. Since the Abscopal effect radiotherapy factors in a phenomenon in which irradiation of a primary lesion with radiation also affects a metastatic lesion which is not directly irradiated, a recommended dose for a primary lesion or a metastatic lesion is displayed, for example. Alternatively, a recommended dose for a primary lesion is displayed and observation of a metastatic lesion is recommended (in other words, radiotherapy is not recommended). The recommended dose may be automatically set based on a total volume of multiple tumors or a primary lesion type, and other biological characteristics of tumors, or may be designated by a user through the input interface 64.

In the foregoing example, it is described that the processing circuitry 61 classifies a tumor area as either a primary lesion or a metastatic lesion based on feature amounts of two or more types of medical image. However, the processing circuitry 61 may also classify a tumor area as either a primary lesion or a metastatic lesion based on feature amounts of a single medical image. For example, the processing circuitry 61 may specify a tumor location and a tumor area based on image feature amounts relating to a tumor area, and may classify the tumor area as either a primary lesion or a metastatic lesion in accordance with result of specification. As the classification method, techniques such as machine learning, data integration, or data mining may be adopted, for example. Herein, the classification method will be described taking a case where the algorithm of machine learning is a DNN as an example.

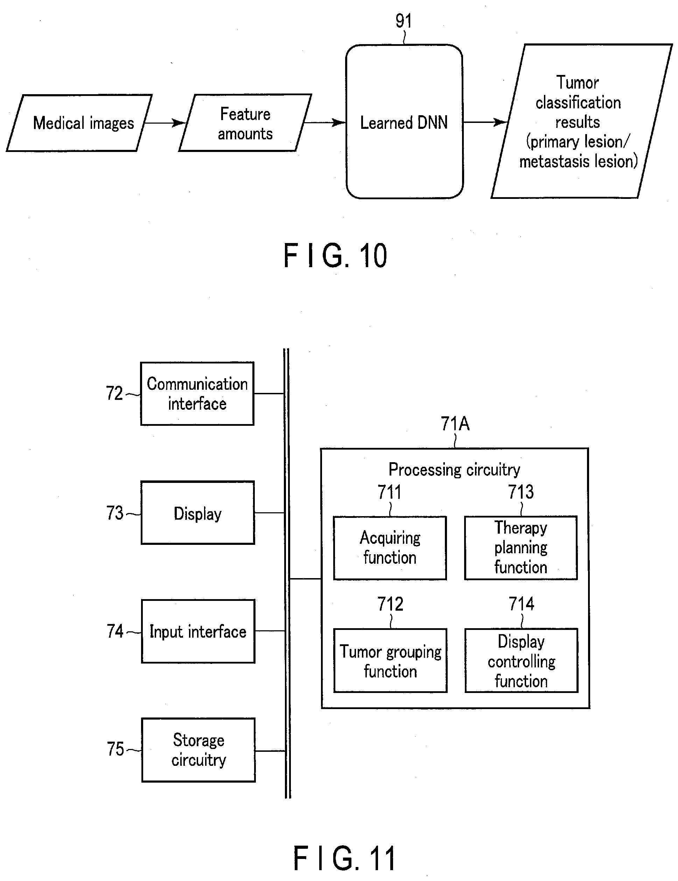

FIG. 10 is a diagram schematically showing an input-output relationship of a learned DNN 91 that classifies a tumor area as either a primary lesion or a metastatic lesion. The learned DNN 91 is a DNN into which a feature amount of a single medical image is input and learned so as to output classification result information including a result of classifying a tumor area as either a primary lesion or a metastatic lesion. The image feature amount and the classification result information are the same as those in the foregoing example.

The medical image may be image data generated by any medical image diagnosis apparatus, such as an X-ray diagnosis apparatus, a CT (X-ray computer tomography imaging) apparatus, an MRI (magnetic resonance imaging) apparatus, a nuclear medical diagnosis apparatus, or an ultrasonic diagnosis apparatus, and the like. For example, the processing circuitry 61 may input an image feature amount relating to a tumor area included in a single medical image into the learned DNN 91, and outputs classification result information obtained by the learned DNN 91. The learned DNN 91 may be a DNN into which feature amounts of medical images are input to learn parameters so as to output only a classification result indicating whether a tumor area is a primary lesion or a metastatic lesion. Furthermore, processing of obtaining image feature amounts relating to a tumor area from a single medical image may be incorporated into the learned DNN 91. Similar to the learned DNN 90 shown in FIG. 4, additional information other than the feature amount may be input into the learned DNN 90. As described above, the additional information may be patient basic information, living body measurement values, genetic information, finding information, etc.

According to the learned DNN 91, it is thus possible to obtain a classification result from a single medical image and to reduce burdens on the patient due to medical imaging and burdens on the processing circuitry 61 due to the classification processing.

The therapy planning apparatus 7 according to a second example produces a therapeutic plan using classification results of tumor areas obtained by the therapy planning support apparatus 6. Hereinafter, the radiotherapy system 1 according to the second example will be described. In the following description, structural elements having substantially the same functions as in the first example will be denoted by the same reference symbols, and duplicate descriptions will be given only where necessary.

As an example, the therapy planning apparatus 7 according to the second example obtains a patient medical image (the medical image marked by markers shown in FIG. 5), and classification result information including a result of classification of a tumor area included in the medical image as either a primary lesion or a metastatic lesion. The therapy planning apparats 7 produces a radiotherapy plan for patient based on the obtained classification result information.

FIG. 11 is a diagram showing a configuration of the therapy planning apparatus 7 included in the radiotherapy system 1 according to the second example. As shown in FIG. 11, the radiotherapy planning apparatus 7 includes processing circuitry 71A, a communication interface 72, a display 73, an input interface 74, and storage circuitry 75.

The processing circuitry 71A includes, as hardware resources, a processor such as a CPU, an MPU, etc. and a memory such as a ROM, a RAM, etc. The processing circuitry 71A executes a program relating to the therapy planning (hereinafter, referred to as "therapy planning program"), to enable an acquiring function 711, a tumor grouping function 712, a therapy planning function 713, and a display controlling function 714.

The processing circuitry 71A that enables the acquiring function 711 acquires a medical image relating to a patient, and classification result information indicating whether or not each of two or more tumor areas included in the medical image is a primary lesion or a metastatic lesion. The medical image may be image data generated by any medical image diagnosis apparatus, such as an X-ray diagnosis apparatus, a CT (X-ray computer tomography imaging) apparatus, an MRI (magnetic resonance imaging) apparatus, a nuclear medical diagnosis apparatus, or an ultrasonic diagnosis apparatus, and the like. The medical image is typically a medical image used to generate classification result information, and specifically a therapy-planning CT image.

The processing circuitry 71A that enables the tumor grouping function 712 sorts a tumor area into one or more groups of different primary lesions, based on the classification result information. Hereinafter, the groups will be referred to as "primary lesion groups".

The processing circuitry 71A that enables the therapy planning function 713 produces a therapeutic plan for each of one or more primary lesion group. The therapeutic plan produced includes a therapeutic plan regarding the primary lesion groups, and a therapeutic plan regarding each tumor area included in the primary lesion groups.

The processing circuitry 71A that enables the display control function 714 displays various types of information via the display 73. Specifically, the processing circuitry 71A displays the therapeutic plan produced by the therapy planning function 713.

The communication interface 72, via wires (not shown) or wirelessly, performs a data communication with the therapy planning CT apparatus 2, the MRI apparatus 3, the nuclear medical diagnosis apparatus 4, the ultrasonic diagnosis apparatus 5, the therapy planning support apparatus 6, and the radiotherapy apparatus 8, which constitute the radiotherapy system 1.

The display 73 displays various types of information through the display controlling function 714. As the display 73, it is possible to use, for example, a CRT display, a liquid crystal display, an organic EL display, an LED display, a plasma display, or any other display known in this technical field as appropriate. The display 73 may be a projector.

The input interface 74 includes an input device and an input interface circuit. The input device receives various types of instructions from a user. A keyboard, a mouse, or switches etc. may be used as the input device. The input interface circuitry supplies an output signal from the input device to the processing circuitry 71A.

The storage circuitry 75 is a storage such as an HDD, an SSD, or an integrated circuit storage which stores various types of information. The storage circuitry 75 may also be a drive assembly or the like which reads and writes various types of information to and from portable storage media, such as a CD-ROM drive, a DVD drive, or a flash memory. For example, the storage circuitry 75 stores the therapy planning program, the therapeutic plan, the therapy-planning CT image, and the other-apparatus images, etc.

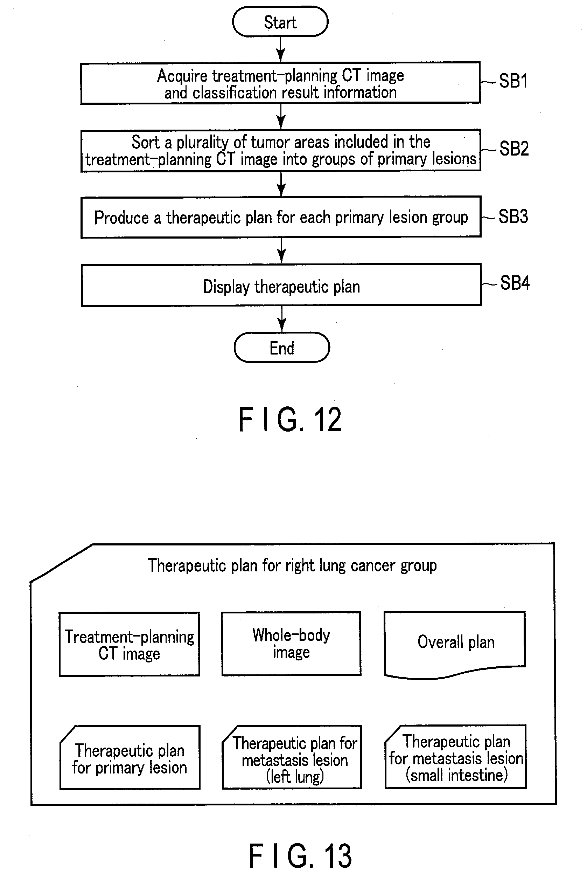

An example of the operation of the therapy planning apparatus 7 according to the present example will be described below. FIG. 12 is a diagram showing a typical workflow realized by execution of the treatment planning program by the processing circuitry 71A. Suppose the processing shown in FIG. 12 is performed after the processing shown in FIG. 3 is finished.

First, the processing circuitry 71A that enables the acquiring function 711 acquires the therapy-planning CT image and the classification result information (step SB1). In step SB1, the processing circuitry 71A obtains the therapy-planning CT image and the classification result information from the therapy planning support apparatus 6 via the communication interface 72, for example. The therapy-planning CT image obtained in step SB1 may be a therapy-planning CT image to which the markers shown in FIG. 5 are placed, or a therapy-planning CT image to which no such markers are placed. The classification result information is generated by the tumor classifying function 612 of the therapy planning support apparatus 6.

After step SB1 is performed, the processing circuitry 71A that enables the tumor grouping function 712 sorts a plurality of tumor areas included in the therapy-planning CT image acquired in step SB1 into groups depending on primary lesions (step SB2). In step SB2, the processing circuitry 71A sorts the plurality of tumor areas into the primary lesion groups based on the classification result information. Specifically, the processing circuitry 71A sorts the plurality of tumor areas into one or more primary lesion groups based on the location information, identification information, and primary lesion information.

For example, suppose the following: for the tumor area RC1 shown in FIG. 5, the location information is "right lung", the identification information is "primary lesion", and the primary lesion information is "primary lesion"; for the tumor area RC2, the location information is "left lung", the identification information is "metastatic lesion", and the primary lesion information is "right lung"; for the tumor area RC3, the location information is "stomach", the identification information is "primary lesion", and the primary lesion information is "primary lesion"; and for the tumor area RC4, the location information is "small intestine", the identification information is "metastatic lesion", and the primary lesion information is "right lung". The tumor area RC1 having the primary lesion information "right lung" is sorted into the primary lesion group "right lung cancer group", and the tumor area RC3 having the primary lesion information "stomach" is sorted into another primary lesion group "stomach cancer group". Then, the tumor areas RC2 and RC4 having the identification information "metastatic lesion" and the primary lesion information "right lung" are sorted into the primary lesion group "right lung cancer group".

After step SB2, the processing circuitry 71A that enables the therapy planning function 713 produces a therapeutic plan for each primary lesion group (step SB3). After step SB3 is performed, the processing circuitry 71A that enables the display controlling function 714 displays the therapeutic plan produced in step SB3 (step SB4).

The processing circuitry 71A produces, for a tumor area classified as a metastatic lesion, a therapeutic plan based on a primary lesion corresponding to the metastatic lesion. In this case, the processing circuitry 71A produces a therapeutic plan in consideration of a lethal dose (or a recommended dose), etc. corresponding to the tumor type, based on a primary lesion specified by the primary lesion information include in the classification result information. For example, the metastatic lesion RC4 shown in FIG. 5 is located at the position of intestine, but it is determined that the primary lesion is specified as "lung" by the primary lesion information included in the classification result information relating to the tumor area. As described in the foregoing example, the lethal dose of a small intestine cancer and that of a lung cancer does not match. In this case, the processing circuitry 71A produces a therapeutic plan in consideration of a lethal dose of the tumor type, and it is thereby possible to perform appropriate radiotherapy for the metastatic lesion RC4.

The processing circuitry 71A may produce a therapeutic plan in which radiation of a primary lesion included in a group is prioritized than a metastatic lesion belonging to the same group. Furthermore, the processing circuitry 71A may produce a therapeutic plan in which at least one of a radiation dose, radiation timing, or the number of radiation is differentiated between a primary lesion and a metastatic lesion belonging to the same group. FIG. 13 is a diagram showing a configuration example of a therapeutic plan of a right lung cancer group. As shown in FIG. 13, the therapeutic plan of the right lung cancer group includes the therapy-planning CT image, the whole-body image, the overall plan, the therapeutic plan of the primary lesion (right lung) RC1, the therapeutic plan of the metastatic lesion (left lung) RC3, and the therapeutic plan of the metastatic lesion (small intestine) RC4. The therapy-planning CT image, the whole-body image, the overall plan, the therapeutic plan for the primary lesion (right lung) RC1, the therapeutic plan for the metastatic lesion (left lung) RC3, and the therapeutic plan for the metastatic lesion (small intestine) RC4 are mutually associated and stored in the storage circuitry 75. The therapy-planning CT image, the whole-body image, the overall plan, the therapeutic plan for the primary lesion (right lung) RC1, the therapeutic plan for the metastatic lesion (left lung) RC3, and the therapeutic plan for the metastatic lesion (small intestine) RC4 can be displayed on the display 73 through selection via the input interface 74, etc.

The therapy-planning CT image is the one obtained in step SB1. The whole-body image is the one generated based on the therapy-planning CT image by the image generating function 614.

The overall plan includes information presenting a guide for the therapeutic plan for all the plurality of tumor areas RC1, RC3, and RC4 included in the right lung cancer group. For example, the processing circuitry 71A determines whether or not the primary lesion and the metastatic lesion belong to the same group satisfies a predetermined Abscopal effect criterion based on the classification result information, and produces a therapeutic plan in which radiation of the primary lesion belonging to the same group is prioritized to radiation of the metastatic lesion belonging to the same group based on the result of determination. The processing circuitry 71A causes the display 73 to display a message recommending Absocpal effect radiotherapy, as shown in FIG. 9. The processing circuitry 71A produces texts, etc., indicating the performance of radiotherapy factoring in the Abscopal effect on the tumor areas RC1, RC3, and RC4, to form the overall plan. Specifically, the processing circuitry 71A determines whether or not the metastatic lesion RC3 satisfies the Abscopal effect criterion in connection with the primary lesion RC1. If it is determined that the metastatic lesion RC3 satisfies the Abscopal effect criterion in connection with the primary lesion RC1, the processing circuitry 71A produces, as the overall plan, a data file of texts, etc. indicating that radiotherapy for the metastatic lesion RC3 is commenced after radiotherapy for the primary lesion RC1 is completed. Alternatively, the processing circuitry 71A may produce, as the overall plan, a data file of texts, etc. indicating that radiotherapy is performed for the metastatic lesion RC3 with a recommended dose, or indicating recommendation of observation (unnecessity of radiotherapy). An overall plan for the metastatic lesion RC4 can be produced in a similar manner. The overall plan may be displayed as a message MS superimposed on the medical image, such as the whole-body image I1, etc. as shown in FIG. 9, or may be displayed simply as a message MS, for example.

The therapeutic plan for the primary lesion RC1 includes a dose distribution, etc. for the primary lesion RC1. The therapeutic plan of the metastatic lesion RC3 includes a dose distribution, etc. for the left lung which is a metastatic lesion. The therapeutic plan for the metastatic lesion RC4) includes a dose distribution, etc. for the small intestine which is a metastatic lesion. For example, the lethal dose for small intestine cancer does not match the lethal dose for lung cancer. For the metastatic lesion RC4, the dose distribution is calculated based on the lethal dose of lung cancer, not small intestine cancer. When the Abscopal effect radiotherapy is performed, since the radiotherapy for the metastatic lesions RC3 an RC4 is performed after the radiotherapy for the primary lesion RC1, it is expected that the metastatic lesions RC3 and RC4 have already been reduced at the time of commencing the radiotherapy for the metastatic lesions RC3 and RC4. Thus, for the metastatic lesions RC3 and RC4, the processing circuitry 71A preferably generates a dose distribution with a recommended dose, or conducts observation (unnecessity of radiotherapy). Each dose distribution is displayed on the display 73 in accordance with a user's instruction to display via the input interface.

When the Abscopal effect radiotherapy is performed, the therapeutic plan for the metastatic lesions RC3 and RC4 may not necessarily be generated along with the therapeutic plan for the primary lesion RC1. For example, the therapeutic plan for the metastatic lesions RC3 and RC4 may be produced after the radiotherapy for the primary lesion RC1 is performed. In this case, the therapy planning CT apparatus 2 may once again perform CT imaging on the patient after the radiotherapy for the primary lesion RC1 is completed, and generate a therapy-planning CT image of the patient. The processing circuitry 71A preferably produces a therapeutic plan based on this newly generated therapy-planning CT image. It is thus possible to produce a therapeutic plan reflecting the reduction of the metastatic lesions RC3 and RC4 due to the Abscopal effect.

Other than the above, the processing circuitry 71A causes the display 73 to display the medical image in which the markers shown in FIG. 5 are placed obtained from the therapy planning support apparatus 6.

Even when the markers are not placed in the medical image obtained from the therapy planning support apparatus 6, it is possible to place markers based on the classification result information obtained from the therapy planning support apparatus 6. For example, the processing circuitry 71A may cause the display 73 to display a medical image in such a manner that whether or not a tumor area is a primary lesion or a metastatic lesion is visually discernible by placing different markers based on the obtained classification result information. Examples of such markers are markers with multiple levels shown in FIG. 5, specifically ⊚ for a primary lesion and O for a metastatic lesion. The processing circuitry 71A may cause the display 73 to display a different marker in accordance with a tumor type or a primary lesion, based on the obtained classification result information so that a tumor type of a tumor area can be visually distinguished. Examples of such markers are markers of different line types shown in FIG. 5; specifically, a solid-line marker for lung cancer and a dotted-line marker for stomach cancer.

Furthermore, similarly to the forgoing example, the processing circuitry 71A may cause the display 73 to display a tumor area included in a same slice of a medical image so that whether a primary lesion or a metastatic lesion can be visually distinguished. Similarly to the foregoing embodiment, the processing circuitry 71A may specify an OAR area included in the medical image and set the shape of a marker indicating the location of the tumor area so that the marker does not overlap the OAR area. The processing circuitry 71A may cause the display 73 to display the set marker and the OAR area.

The above is the description of an operation example of the therapy planning.

According to the present example, an appropriate therapeutic plan in accordance with a tumor can be produced based on the classification result information obtained for the tumor area included in the metical image. For example, even if the tumor area included in the medical image is located at the position of left lung, if the classification result information obtained for the tumor area indicates a metastatic lesion originating from stomach cancer (in other words, the identification information indicates "metastatic lesion" and the metastasis information indicates "stomach") it is possible to produce a therapeutic plan in consideration of a lethal dose for a metastatic lesion originating from stomach cancer. It is possible to sort the tumor areas into different primary lesion groups, and to produce a therapeutic plan for each primary lesion group. It is thereby possible to produce an overall therapeutic plan for a primary lesion and its metastatic lesions as the elements of the same primary lesion group, unlike in the case where individual therapeutic plans are produced for a primary lesion and a metastatic lesion. Thus, a user can have an opportunity to produce a therapeutic plan for radiotherapy factoring in the Abscopal effect. Collective management of therapeutic plans factoring in the Abscopal effect can be realized through the storage of therapeutic plans for a primary lesion, and those for its metastatic lesion being associated with each other.

In the first and second examples, the classification as either a primary lesion or a metastatic lesion is made by the therapy planning support apparatus 6, and the therapeutic plan is produced by the therapy planning apparatus 7; however, these acts of processing may be performed only by the therapy planning apparatus 7. Hereinafter, the radiotherapy system 1 according to a third example will be described below. In the following description, structural elements having substantially the same functions as in the first and second examples will be denoted by the same reference symbols, and duplicate descriptions will be given only where necessary.

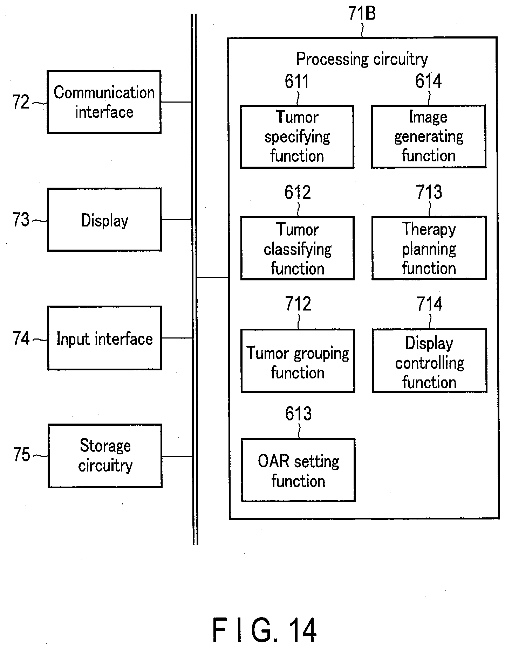

FIG. 14 is a diagram showing a configuration of the therapy planning apparatus 7 included in the radiotherapy system 1 according to the third example. As shown in FIG. 14, the therapy planning apparatus 7 includes the processing circuitry 71B, the communication interface 72, the display 73, the input interface 74, and the storage circuitry 75. The processing circuitry 71B includes, as hardware resources, a processor such as a CPU, an MPU, etc. and a memory such as a ROM, a RAM, etc. The processing circuitry 71B executes the therapy planning program to enable the tumor specifying function 611, the tumor classifying function 612, and the OAR setting function 613, the image generating function 614, the tumor grouping function 712, the therapy planning function 713, and the display controlling function 714. The descriptions of the hardware resources and functions are omitted.

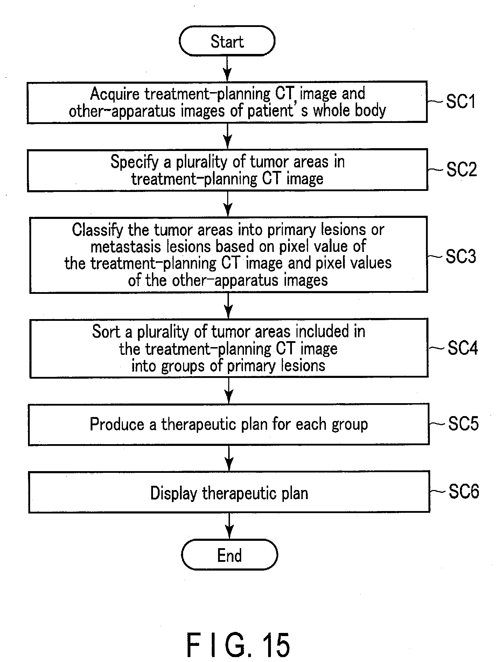

An example of the operation of the radiotherapy planning apparatus 7 according to the present example will be described below. FIG. 15 is a diagram showing a typical workflow realized by execution of the treatment planning program by the processing circuitry 71B according to the third example.

First, the processing circuitry 71B acquires a therapy-planning CT image and other-apparatus images of the patient's whole body (step SC1). The processing in step SC1 is similar to that in step SA1.

After step SC1 is performed, the processing circuitry 71B that enables the tumor specifying function 611 specifies a plurality of tumor areas in the therapy-planning CT image acquired in step SC1 (step SC2). The processing in step SC2 is similar to that in step SA2.

After step SC2 is performed, the processing circuitry 71B that enables the tumor classifying function 612 classifies each tumor area specified in step SC2 as either a primary lesion or a metastatic lesion based on a pixel value of the therapy-planning CT image and pixel values of the other-apparatus images (step SC3). The processing in step SC3 is similar to that in step SA3. In step SC3, the processing circuitry 71B generates classification result information for each tumor area. In this case, the processing circuitry 71B classifies each tumor area as either a primary lesion or a metastatic lesion based on the pixel value of the therapy-planning CT image and the pixel values of the other-apparatus images.

After step SC3 is performed, the processing circuitry 71B that enables the tumor grouping function 712 sorts a plurality of tumor areas included the therapy-planning CT image into primary lesion groups (step SC4). The processing in step SC4 is similar to that in step SB2.

After step SC4 is performed, the processing circuitry 71B that enables the therapy planning function 713 produces a therapeutic plan for each primary lesion group (step SC5). The processing in step SC5 is similar to that in step SB3.

After step SC5 is performed, the processing circuitry 71B that enables the display controlling function 714 displays the therapeutic plan produced in in step SC5 (step SC6). The processing in step SC6 is similar to that in step SB4.

In the first to third examples described above, the radiotherapy system 1 includes the therapy-planning CT apparatus 2; however, the radiotherapy system 1 may include a general-purpose X-ray computer tomography imaging apparatus not dedicated for therapy planning, and a CT image generated by this general-purpose X-ray computer tomography imaging apparatus may be e as a therapy-planning CT image.

The above is a description of an operation example of the therapy planning.

Moreover, the classification as either a primary lesion or a metastatic lesion (obtaining of a classification result information) and the production of a therapeutic plan based on the classification result information (a therapeutic plan for each primary lesion group) can be achieved.

According to at least one of the foregoing examples, it is possible to classify a tumor area as either a primary lesion or a metastatic lesion in the therapy planning.

The term "processor" used in the above explanation indicates, for example, a circuit, such as a CPU, a GPU, or an Application Specific Integrated Circuit (ASIC), and a programmable logic device (for example, a Simple Programmable Logic Device (SPLD), a Complex Programmable Logic Device (CPLD), and a Field Programmable Gate Array (FPGA)). The processor realizes its function by reading and executing the program stored in the storage circuitry. Instead of storing a program on the memory circuitry, the program may be directly integrated into the circuitry of the processor. In this case, the function is activated by reading and executing the program integrated into the circuitry. The function corresponding to the program may be realized by a combination of logic circuits, and not by execution of the program. Each processor of the present embodiment is not limited to a case where each processor is configured as a single circuit; a plurality of independent circuits may be combined into one processor to realize the function of the processor. Furthermore, a plurality of constituent elements shown in FIGS. 1, 2, 11, and 14 may be integrated into a single processor to realize their functions.

## Claims

1. A radiotherapy system (1) comprising processing circuitry (61, 71A, 71B), the processing circuitry (61, 71A, 71B)configured to:
obtain classification result information including a result of classification of a tumor area (RCn) included in a medical image of a patient as either a primary lesion or a metastatic lesion;
sort the tumor area (RCn) into one or more groups of primary lesion based on the classification result information; and
produce a radiotherapy plan in which radiation of a primary lesion included in the group is prioritized to radiation of a metastatic lesion belonging to the same group.

2. The radiotherapy system of claim 1, wherein
the processing circuitry (61, 71A, 71B) is configured to generate the radiotherapy plan in which at least one of a radiation dose, radiation timing, and the number of performed radiation is different between the primary lesion and the metastatic lesion belong to the same group.

3. The radiotherapy system of claim 2, wherein
the processing circuitry (61, 71A, 71B)is configured to determine whether or not the primary lesion and the metastatic lesion belong to the same group satisfies a predetermined Abscopal effect criterion, and to produce, in accordance with a result of the determining, the radiotherapy plan in which radiation of a primary lesion included in the group is prioritized to radiation of a metastatic lesion belonging to the same group.

4. The radiotherapy system of claim 1, wherein
the classification result information has primary lesion information specifying a primary lesion for a tumor area (RCn) classified as a metastatic lesion, and
the processing circuitry (61, 71A, 71B)is configured to produce the radiotherapy plan based on a primary lesion specified by the primary lesion information.

5. The radiotherapy system of claim 1, wherein
the processing circuitry (61, 71A, 71B)is configured to cause a display (63, 73) to display the tumor area (RCn) in a way which provides a visual distinction between a primary lesion or a metastatic lesion based on the classification result information.

6. The radiotherapy system of claim 5, wherein
the processing circuitry (61, 71A, 71B) is configured to cause the display (63, 73) to display the tumor area (RCn) in a way which provides a visual distinction of a tumor type of the tumor area (RCn) based on the classification result information.

7. The radiotherapy system of claim 5, wherein
the processing circuitry (61, 71A, 71B) is configured to cause the display (63, 73) to display tumor areas (RCn) included in a same slice of the medical image in a way which provides a visual distinction between a primary lesion and a metastatic lesion based on the classification result information.

8. The radiotherapy system of claim 5, wherein
the processing circuitry is configured to determine whether or not the primary lesion and the metastatic lesion belong to a same group satisfies a predetermined Abscopal effect criterion, and may cause the display to display a message recommending Abscopal effect radiotherapy in accordance with the determination result.

9. The radiotherapy system of claim 1, wherein
the processing circuitry (61, 71A, 71B) is configured to specify an organ-at risk "OAR" area included in the medical image, and set a shape or a marker (M5) indicating a location of the tumor area so that the marker (M5) does not overlap the OAR area, and may cause the display to display the marker of the shape.

10. The radiotherapy system of claim 1, wherein
the processing circuitry (61, 71A, 71B) is configured to:
acquire first and second medical images of a patient generated by first and second medical modalities (2, 3, 4, 5) that generate medical images by different principles of imaging;
specify at least one tumor area (RCn) of the patient based at least one of the first and second medical images; and
classify the tumor area (RCn) as either a primary lesion or a metastatic lesion based on an image feature amount of the first medical image relating to the tumor area and a second image feature amount of the second medical image relating to the same tumor area (RCn) to obtain the classification result information.

11. The radiotherapy system of claim 10, wherein
the processing circuitry (61, 71A, 71B)is configured to specify a tumor location and a tumor type based on the first and second image feature amounts, and to classify the tumor area (RCn) as either a primary lesion or a metastatic lesion based on a result of the specifying.

12. The radiotherapy system of claim 10, wherein
the processing circuitry (61, 71A, 71B) is configured to have a learned model learned to obtain the classification result information from an input of the first image feature amount of the first medical image and the second image feature amount of the second medical image, and to input the first and second image feature amounts relating to the tumor area (RCn) of the patient to the learned model, and output the classification result information obtained by the learned model.

13. The radiotherapy system of claim 11, wherein
if the tumor area (RCn) is classified as a metastatic lesion, the processing circuit (61, 71A, 71B) is configured to include information specifying a primary lesion corresponding to the metastatic lesion in the classification result information

14. A therapy planning method comprising:
acquiring classification result information including a result of classification of a tumor area (RCn) included in a medical image of a patient as either a primary lesion or a metastatic lesion;
sorting the tumor area (RCn) into one or more groups of primary lesion based on the classification result information; and
producing a radiotherapy plan in which radiation of a primary lesion included in the group is prioritized to radiation of a metastatic lesion belonging to the same group.

15. The therapy planning method of claim 14, comprising:
acquiring first and second medical images of a patient generated by first and second medical modalities (2, 3, 4, 5) that generate medical images by different principles of imaging;
specifying at least one tumor area (RCn) of the patient based at least one of the first and second medical images; and
classifying the tumor area (RCn) as either a primary lesion or a metastatic lesion based on a first image feature amount of the first medical image relating to the tumor area (RCn) and a second image feature amount of the second medical image relating to the same tumor area (RCn) to acquire the classification result information.

## Patentansprüche

1. Strahlentherapiesystem (1), umfassend eine Verarbeitungsschaltung (61, 71A, 71B), wobei die Verarbeitungsschaltung (61, 71A, 71B) konfiguriert ist, zum:
Erhalten von Informationen über das Klassifizierungsergebnis, einschließlich eines Ergebnisses der Klassifizierung eines Tumorbereichs (RCn), der in einem medizinischen Bild eines Patienten als entweder eine primäre Läsion oder eine metastatische Läsion eingeschlossen ist,
Sortieren des Tumorbereichs (RCn) in eine oder mehrere Gruppen von primären Läsionen auf der Grundlage der Klassifizierungsergebnisinformationen; und
Erstellen eines Strahlentherapieplans, in dem die Bestrahlung einer primären Läsion, die in der Gruppe eingeschlossen ist, gegenüber der Bestrahlung einer metastatischen Läsion, die zur selben Gruppe gehört, priorisiert wird.

2. Strahlentherapiesystem nach Anspruch 1, wobei
die Verarbeitungsschaltung (61, 71A, 71B) konfiguriert ist, um den Strahlentherapieplan zu erzeugen, in dem mindestens eines von einer Strahlendosis, einem Bestrahlungszeitpunkt und der Anzahl der durchgeführten Bestrahlungen zwischen der primären Läsion und der metastatischen Läsion, die zur selben Gruppe gehören, unterschiedlich ist.

3. Strahlentherapiesystem nach Anspruch 2, wobei
die Verarbeitungsschaltung (61, 71A, 71B) konfiguriert ist, um zu bestimmen, ob die Bedingung, dass die primäre Läsion und die metastatische Läsion zur gleichen Gruppe gehören oder nicht, ein vorbestimmtes Abscopal-Effekt-Kriterium erfüllt, und um in Übereinstimmung mit einem Ergebnis der Bestimmung den Strahlentherapieplan zu erstellen, in dem die Bestrahlung einer primären Läsion, die in der Gruppe eingeschlossen ist, gegenüber der Bestrahlung einer metastatischen Läsion, die zu derselben Gruppe gehört, priorisiert wird.

4. Strahlentherapiesystem nach Anspruch 1, wobei
die Klassifizierungsergebnisinformationen primäre Läsionsinformationen enthalten, die eine primäre Läsion für einen als metastatische Läsion klassifizierten Tumorbereich (RCn) angeben, und
die Verarbeitungsschaltung (61, 71A, 71B) konfiguriert ist, um den Strahlentherapieplan auf der Grundlage einer primären Läsion zu erstellen, die durch die Informationen zur primären Läsion angegeben wird.

5. Strahlentherapiesystem nach Anspruch 1, wobei
die Verarbeitungsschaltung (61, 71A, 71B) konfiguriert ist, um eine Anzeige (63, 73) zu veranlassen, den Tumorbereich (RCn) in einer Weise anzuzeigen, die eine visuelle Unterscheidung zwischen einer primären Läsion oder einer metastatischen Läsion auf der Grundlage der Klassifizierungsergebnisinformationen bereitstellt.

6. Strahlentherapiesystem nach Anspruch 5, wobei
die Verarbeitungsschaltung (61, 71A, 71B) konfiguriert ist, um die Anzeige (63, 73) zu veranlassen, den Tumorbereich (RCn) auf eine Weise anzuzeigen, die eine visuelle Unterscheidung eines Tumortyps des Tumorbereichs (RCn) basierend auf den Klassifizierungsergebnisinformationen bereitstellt.

7. Strahlentherapiesystem nach Anspruch 5, wobei
die Verarbeitungsschaltung (61, 71A, 71B) konfiguriert ist, um die Anzeige (63, 73) zu veranlassen, Tumorbereiche (RCn), die in einer gleichen Scheibe des medizinischen Bildes eingeschlossen sind, auf eine Weise anzuzeigen, die eine visuelle Unterscheidung zwischen einer primären Läsion und einer metastatischen Läsion auf der Basis der Klassifikationsergebnisinformationen bereitstellt.

8. Strahlentherapiesystem nach Anspruch 5, wobei
die Verarbeitungsschaltung konfiguriert ist, um zu bestimmen, ob die Bedingung, dass die primäre Läsion und die metastatische Läsion zu einer gleichen Gruppe gehören oder nicht, ein vorbestimmtes Abscopal-Effekt-Kriterium erfüllt, und kann die Anzeige veranlassen, eine Nachricht anzuzeigen, die eine Abscopal-Effekt-Strahlentherapie in Übereinstimmung mit dem Bestimmungsergebnis empfiehlt.

9. Strahlentherapiesystem nach Anspruch 1, wobei
die Verarbeitungsschaltung (61, 71A, 71B) konfiguriert ist, um einen Organ-at-Risk "OAR"-Bereich anzugeben, der in dem medizinischen Bild eingeschlossen ist, und um eine Form oder eine Markierung (M5) zu setzen, die eine Stelle des Tumorbereichs anzeigt, so dass die Markierung (M5) den OAR-Bereich nicht überlappt, und kann die Anzeige veranlassen, die Markierung der Form anzuzeigen.

10. Strahlentherapiesystem nach Anspruch 1, wobei
die Verarbeitungsschaltung (61, 71A, 71B) konfiguriert ist, zum:
Erfassen von ersten und zweiten medizinischen Bildern eines Patienten, die von ersten und zweiten medizinischen Modalitäten (2, 3, 4, 5) erzeugt wurden, die medizinische Bilder nach unterschiedlichen Prinzipien der Bildgebung erzeugen;
Spezifizieren mindestens eines Tumorbereichs (RCn) des Patienten auf der Grundlage mindestens eines der ersten und zweiten medizinischen Bilder; und
Klassifizieren des Tumorbereichs (RCn) entweder als primäre Läsion oder als metastatische Läsion, basierend auf einem Bildmerkmalswert des ersten medizinischen Bildes, der sich auf den Tumorbereich bezieht, und auf einem zweiten Bildmerkmalswert des zweiten medizinischen Bildes, der sich auf denselben Tumorbereich (RCn) bezieht, um die Klassifikationsergebnisinformationen zu erhalten.

11. Strahlentherapiesystem nach Anspruch 10, wobei
die Verarbeitungsschaltung (61, 71A, 71B) konfiguriert ist, um eine Tumorposition und einen Tumortyp basierend auf dem ersten und zweiten Bildmerkmalswert zu spezifizieren und den Tumorbereich (RCn) entweder als primäre Läsion oder als metastatische Läsion zu klassifizieren, basierend auf einem Spezifizierungsergebnis.

12. Strahlentherapiesystem nach Anspruch 10, wobei
die Verarbeitungsschaltung (61, 71A, 71B) konfiguriert ist, um ein erlerntes Lernmodell aufzuweisen, um die Klassifizierungsergebnisinformationen aus einer Eingabe des ersten Bildmerkmalswerts des ersten medizinischen Bildes und des zweiten Bildmerkmalswerts des zweiten medizinischen Bildes zu erhalten, und um den ersten und zweiten Bildmerkmalswert, die sich auf den Tumorbereich (RCn) des Patienten beziehen, in das Lernmodell einzugeben, und um die durch das gelernte Modell erhaltenen Klassifizierungsergebnisinformationen auszugeben.

13. Strahlentherapiesystem nach Anspruch 11, wobei
wenn der Tumorbereich (RCn) als metastatische Läsion klassifiziert wird, die Verarbeitungsschaltung (61, 71A, 71B) konfiguriert ist, um Informationen, die eine primäre Läsion spezifizieren, die der metastatischen Läsion entspricht, in die Klassifizierungsergebnisinformationen einzuschließen.

14. Therapieplanungsverfahren, umfassend:
Erfassen von Klassifizierungsergebnisinformationen einschließlich eines Ergebnisses der Klassifizierung eines Tumorbereichs (RCn), der in einem medizinischen Bild eines Patienten entweder als primäre Läsion oder als metastatische Läsion eingeschlossen ist,
Sortieren des Tumorbereichs (RCn) in eine oder mehrere Gruppen von primären Läsionen auf der Grundlage der Klassifizierungsergebnisinformationen; und
Erstellen eines Strahlentherapieplans, in dem die Bestrahlung einer primären Läsion, die in der Gruppe eingeschlossen ist, gegenüber der Bestrahlung einer metastatischen Läsion, die zur gleichen Gruppe gehört, priorisiert wird.

15. Therapieplanungsverfahren nach Anspruch 14, umfassend:
Erfassen von ersten und zweiten medizinischen Bildern eines Patienten, die durch die ersten und zweiten medizinischen Modalitäten (2, 3, 4, 5) erzeugt wurden, die medizinische Bilder nach unterschiedlichen Prinzipien der Bildgebung erzeugen;
Spezifizieren mindestens eines Tumorbereichs (RCn) des Patienten auf der Grundlage mindestens eines der ersten und zweiten medizinischen Bilder; und
Klassifizieren des Tumorbereichs (RCn) als entweder eine primäre Läsion oder eine metastatische Läsion auf der Grundlage eines ersten Bildmerkmalswerts des ersten medizinischen Bildes, der sich auf den Tumorbereich (RCn) bezieht, und eines zweiten Bildmerkmalswerts des zweiten medizinischen Bildes, der sich auf denselben Tumorbereich (RCn) bezieht, um die Klassifizierungsergebnisinformationen zu erfassen.

## Revendications

1. Système de radiothérapie (1) comprenant un circuit de traitement (61, 71A, 71B), le circuit de traitement (61, 71A, 71B) étant configuré pour :
obtenir des informations de résultat de classification incluant un résultat de classification d'une région de tumeur (RCn) incluse dans une image médicale d'un patient en tant qu'une lésion primaire ou une lésion métastatique,
trier la région de la tumeur (RCn) en un ou plusieurs groupes de lésion primaire sur la base des informations de résultat de classification, et
produire un plan de radiothérapie dans lequel l'irradiation d'une lésion primaire incluse dans le groupe est priorisée par rapport à une irradiation d'une lésion métastatique appartenant au même groupe.

2. Système de radiothérapie selon la revendication 1, dans lequel
le circuit de traitement (61, 71A, 71B) est configuré pour générer le plan de radiothérapie dans lequel au moins l'un parmi une dose d'irradiation, un calendrier d'irradiation et le nombre d'irradiations exécutées est différent entre la lésion primaire et la lésion métastatique appartenant au même groupe.

3. Système de radiothérapie selon la revendication 2, dans lequel
le circuit de traitement (61, 71A, 71B) est configuré pour déterminer si le fait que la lésion primaire et la lésion métastatique font partie du même groupe satisfait ou non à un critère d'effet abscopal prédéterminé, et pour produire, en fonction d'un résultat de la détermination, le plan de radiothérapie dans lequel l'irradiation d'une lésion primaire incluse dans le groupe reçoit la priorité par rapport à l'irradiation d'une lésion métastatique appartenant au même groupe.

4. Système de radiothérapie selon la revendication 1, dans lequel
les informations de résultat de classification comportent des informations de lésion primaire spécifiant une lésion primaire pour une région de tumeur (RCn) classifiée comme lésion métastatique, et
le circuit de traitement (61, 71A, 71B) est configuré pour produire le plan de radiothérapie sur la base d'une lésion primaire spécifiée par les informations relatives à la lésion primaire.

5. Système de radiothérapie selon la revendication 1, dans lequel
le circuit de traitement (61, 71A, 71B) est configuré pour amener un affichage (63, 73) à afficher la région de la tumeur (RCn) de façon à fournir une distinction visuelle entre une lésion primaire ou une lésion métastatique sur la base des informations de résultat de classification.

6. Système de radiothérapie selon la revendication 5, dans lequel
le circuit de traitement (61, 71A, 71B) est configuré pour amener l'affichage (63, 73) à afficher la région de la tumeur (RCn) de façon à fournir une distinction visuelle d'un type de tumeur de la région de tumeur (RCn) sur la base des informations de résultat de classification.

7. Système de radiothérapie selon la revendication 5, dans lequel
le circuit de traitement (61, 71A, 71B) est configuré pour amener l'affichage (63, 73) à afficher des régions de la tumeur (RCn) incluses dans une même tranche de l'image médicale de façon à fournir une distinction visuelle entre une lésion primaire et une lésion métastatique sur la base des informations de résultat de classification.

8. Système de radiothérapie selon la revendication 5, dans lequel
le circuit de traitement est configuré pour déterminer si le fait que la lésion primaire et la lésion métastatique appartiennent à un même groupe satisfait ou non à un critère d'effet abscopal prédéterminé, et peut amener l'affichage à afficher un message recommandant une radiothérapie avec un effet abscopal selon le résultat de la détermination.

9. Système de radiothérapie selon la revendication 1, dans lequel
le circuit de traitement (61, 71A, 71B) est configuré pour spécifier une région d'organe à risque « OAR » incluse dans l'image médicale, et pour placer une forme ou un repère (M5) indiquant un emplacement de la région de la tumeur, de sorte que le repère (M5) ne chevauche pas la région de l'OAR, et peut amener l'affichage à afficher le repère ou la forme.

10. Système de radiothérapie selon la revendication 1, dans lequel
le circuit de traitement (61, 71A, 71B) est configuré pour :
acquérir des première et deuxième images médicales d'un patient générées par des première et deuxième modalités médicales (2, 3, 4, 5) qui génèrent des images médicales selon différents principes d'imagerie,
spécifier au moins une région de tumeur (RCn) du patient sur la base d'au moins une de la première et de la deuxième image médicale, et
classifier la région de tumeur (RCn) en tant que lésion primaire ou lésion métastatique sur la base d'une quantité de caractéristique d'image de la première image médicale liée à la région de tumeur et d'une deuxième quantité de caractéristique d'image de la deuxième image médicale liée à la même région de tumeur (RCn) pour obtenir les informations de résultat de la classification.

11. Système de radiothérapie selon la revendication 10, dans lequel
le circuit de traitement (61, 71A, 71B) est configuré pour spécifier un emplacement de tumeur et un type de tumeur sur la base des première et deuxième quantités de caractéristique d'image, et pour classifier la région de tumeur (RCn) en tant que lésion primaire ou que lésion métastatique sur la base du résultat de la spécification.

12. Système de radiothérapie selon la revendication 10, dans lequel
le circuit de traitement (61, 71A, 71B) est configuré pour présenter un modèle entraîné pour obtenir les informations de résultat de classification à partir d'une entrée de la première quantité de caractéristique d'image de la première image médicale et de la deuxième caractéristique d'image de la deuxième image médicale, et pour entrer les première et deuxième quantités de caractéristique d'image liées à la région de tumeur (RCn) du patient dans le modèle entraîné, et pour délivrer les informations de résultat de classification obtenues par le modèle entraîné.

13. Système de radiothérapie selon la revendication 11, dans lequel
si la région de tumeur (RCn) est classée comme une lésion métastatique, le circuit de traitement (61, 71A, 71B) est configuré pour inclure des informations spécifiant une lésion primaire correspondant à la lésion métastatique dans les informations de résultat de classification.

14. Procédé de planification de thérapie comprenant :
l'acquisition d'informations de résultat de classification incluant un résultat de classification d'une région de tumeur (RCn) incluse dans une image médicale d'un patient en tant que lésion primaire ou lésion métastatique,
le tri de la région de la tumeur (RCn) en un ou plusieurs groupes de lésion primaire sur la base des informations de résultat de classification, et
la production d'un plan de radiothérapie dans lequel l'irradiation d'une lésion primaire incluse dans le groupe reçoit la priorité par rapport à l'irradiation d'une lésion métastatique appartenant au même groupe.

15. Procédé de planification de thérapie selon la revendication 14, comprenant :
l'acquisition de première et deuxième images médicales d'un patient générées par des première et deuxième modalités médicales (2, 3, 4, 5) qui génèrent des images médicales selon différents principes d'imagerie,
la spécification d'au moins une région de tumeur (RCn) du patient sur la base d'au moins une de la première et de la deuxième image médicale, et
la classification de la région de tumeur (RCn) en tant que lésion primaire ou lésion métastatique sur la base d'une première quantité de caractéristique d'image de la première image médicale liée à la région de tumeur (RCn) et d'une deuxième quantité de caractéristique d'image de la deuxième image médicale liée à la même région de tumeur (RCn) pour acquérir les informations de résultat de classification.
